# EUROPEAN PATENT SPECIFICATION

(11) **EP 1 858 410 B1**
(45) Date of publication and mention of the grant of the patent: **17.12.2014**
(21) Application number: 06736586.6
(22) Date of filing: 01.03.2006
(51) Int. Cl.: A61B 5/00, A61B 5/08, A61B 5/107

(54) **REUSABLE INDUCTIVE TRANSDUCER FOR MEASURING RESPIRATION**
WIEDERVERWENDBARER INDUKTIVER WANDLER ZUR MESSUNG DER ATMUNG
TRANSDUCTEUR INDUCTIF REUTILISABLE POUR LA MESURE DE LA RESPIRATION

(30) Priority: 01.03.2005 US 657953 P
(43) Date of publication of application: 28.11.2007
(73) Proprietor: Pro-Tech Services, Inc., Mukilteo, WA 98275 (US)
(72) Inventor: LINVILLE, David J., Bothell, Washington 98021 (US)
(74) Representative: Damen, Daniel Martijn
(86) International application number: PCT/US2006/007293
(87) International publication number: WO 2006/112956

(56) References cited:
- US-A- 4 817 625
- US-A- 5 913 830
- US-A- 5 913 830
- US-A- 6 142 953
- US-A1- 2002 032 388
- US-A1- 2002 032 388

## Description

### FIELD OF INVENTION

The present invention relates generally to transducers for use in the medical field for physiological patient monitoring. In particular, the invention relates to an extensible respiratory inductance plethysmograph transducer for use in respiratory monitoring to receive signals representative of patient breathing. The transducer of the present invention has at least one conductor woven directly into an extensible material, the conductor having a number and orientation of inductive turns that improves the transducer expandability and the electrical performance over the prior art of sensors for receiving respiratory signals.

### BACKGROUND OF THE INVENTION

Monitoring respiration inductively, known in the art as respiratory inductance plethysmography (RIP), is a highly desirable and superior respiratory monitoring technology over prior art technologies for respiratory monitoring. U.S. Patent No. 4,308,872 to Watson et al. first disclosed RIP in 1982 in the form of a non-invasive apparatus for monitoring respiration without signal polarity problems and without requiring the use of dangerous materials such as mercury. Unlike the piezo technology of the prior art, RIP can also be used in a quantitative function, and because it measures cross-sectional area and not circumference, RIP can be calibrated to approximate respiratory volume accurately. While the benefits of RIP technology are numerous, significant drawbacks have kept it from widespread use.

RIP uses two inductive transducers, each in the form of a conductive loop, and a means to measure their inductance, which in combination provide an electronic signal indicative of the cross-sectional area of the torso segment about which the transducers are looped (e.g., an abdominal or thoracic segment). A change in the inductance of the conductive loop provides a measure of change for the cross sectional area encircled. Changes in inductance that occur with changes in the cross-sectional area of the torso segment due to breathing reflect the respiration activity of a patient. The conductive loop is connected to an electronic monitoring device, which includes circuitry that reliably and accurately measures changes in the inductance of the conductive loop mounted on the torso segment.

The use of an inductive sensor that circumscribes the torso has been found to have certain inherent disadvantages. Non-invasive respiration inductive sensors are usually only semi-quantitative and are subject to signal artifact due to body movement, changes in sleeping position, physical displacement, physical deformation, changes in relative calibration of the chest and abdominal compartments, electrical interference by the chest transducer to the abdominal transducer (and vice-versa), and electrical interference from external electron-magnetic fields including electrical magnetic properties of the torso.

RIP transducers are comprised of one or more conductor segments attached in an extensible way to an extensible substrate. The change in inductance measured is then used to determine respiratory effort and airflow. When one transducer is placed around the abdomen, and another is placed around the chest, respiratory volume can be accurately estimated. RIP is highly desirable over other technologies used to measure respiratory effort because it does not change polarity.

The technology most widely used in the field for measuring cross-sectional area for respiration monitoring is the use of piezo belts, which are inexpensive to produce and easy to use. Piezo belts use a piezoelectric element to generate an electric signal from the mechanical deformation caused by changes in belt circumference. Those signals can be used to infer respiratory effort, but the sensor element is small in comparison to the circumference of the torso, and is subject to localized distortions. These distortions can generate a signal of reverse polarity, which is not indicative of respiratory effort and forces a care provider to determine when the piezo belts are, and are not, functioning correctly. These polarity shifts, referred to as false paradox, can give incorrect indications of respiratory distress, and are a source of error artifact. The relative high cost of RIP transducers in comparison with low cost piezo transducers keeps the less accurate technology in wide use in the field.

While RIP's advantages are widely known and accepted, it has not been widely used because of the high cost of production and ownership. The RIP transducers of the prior art are typically comprised of one or more conductors attached to, or sandwiched between, layers of elastic or non-elastic substrates in geometric patterns, such as saw-tooth or sinusoidal patterns, along the plane of the substrate using a detachable connection device to close the loop around a body or body part to be measured.

Using laminations, U.S. Pat. No. 5,301,678 and U.S. Pat. No. 4,807,640, both to Watson, et al., or stitching, U.S. Pat. No. 4,308,872 also to Watson, et al., to hold a conductor to an elastic substrate is inherently limiting to the flexibility and durability of the sensor. Initially, large numbers of sizes were used to overcome the fundamental constraints of the devices, but later more complicated designs using repeating geometries came into use, such as U.S. Pat. No.4,817,625 and U.S. Pat. No. 5,913,830, both to Miles. These designs use multiple conductor segments or complicated mechanics as in U.S. Pat. No. 6,142,953 to eliminate the need for completely encompassing a body or body part, but further increase manufacturing complications and associated costs while decreasing the accuracy of measured signals.

U.S. Pat. No. 4,308,872 to Watson et al. discloses an apparatus for monitoring respiration having a tubular stretch bandage in the form of a long sleeveless sweater worn closely fitted over the torso of a patient. A conductor is attached in a number of turns around the sweater from an area for covering the lower abdomen to the upper chest, and so will provide a measure of area averaged over the entire torso. More turns may be placed over one portion of the torso and fewer over other portions, if it is desired to give greater weight to changes in area of one portion of the torso relative to others. The multi-turn loop is closed by a vertical section returning to the starting point. Both ends of the loop are electrically connected to an electronic circuit module, which is located on the patient's lower side. In another embodiment, the monitoring apparatus includes two elastic tubes located about the upper chest and the lower abdomen of the patient. Conductors are mounted in a single turn loop circumferentially of tubes. Snap fasteners are provided for holding the band together. While these embodiments teach a stretchable transducer for monitoring respiration, the transducer is limited in the degree to which it can stretch, thus limiting usefulness on a variety of differently sized patients.

U.S. Pat. No. 4,452,252 to Sackner discloses a method for monitoring cardiopulmonary events using an extensible conductor looped in close encircling relation about the neck of a subject to obtain a signal indicative of the inductance of the loop that correlates with a cross-sectional area enclosed by the loop. Changes in the cross-sectional area of the neck occur with cardiopulmonary events, such as each carotid pulse, and can be observed by monitoring the inductance signal obtained. The best mode is provided as disposing an extensible electrically conductive loop supported in "any suitable fashion on an elastic tube or the like" about the neck. The conductive loop is rendered extensible by forming the loop in alternating "up and down looplets" advancing in a plane. A transducer of this type is limited by complicated and expensive methods of manufacturing, and transducer durablility.

U.S. Pat. No. 4,807,640 to Watson et al., entitled "Stretchable Band-Type Transducer Particularly Suited For Respiration Monitoring Apparatus" discloses a monitoring apparatus having a conductor, which is supported on a strip of woven fabric securable about a patient's torso. The fabric strip is stitched under tension by a plurality of longitudinally extending elastic stitches such that when the tension in the strip is released, the fabric becomes bunched or puckered along its entire length. An insulated wire conductor is stitched to one side of the fabric in a zigzag pattern. The stretching of the fabric in a longitudinal direction is accommodated by the puckers or folds with corresponding extension of the wire being accommodated by a widening and flattening of the saw tooth pattern. In use, the length of the band in its unstretched condition should be less than the circumference of the encircled portion of the torso of the patient such that the band may be stretched for a snug fit. To accommodate connection of the wire to the monitoring apparatus, the conductor is secured to the fabric such that both ends of the conductor terminate at a common location along a longitudinal edge of the band. The ends of the conductor are soldered to connecting pins which are then secured in shrink tubing such that the tips of the connecting pins are exposed. The shrink tubing is stapled to the ends of the band. The conductors are then secured to a monitoring device. However, while this transducer provides an improvement over prior art, it has been found to be inherently limiting to the flexibility and durability of the transducer in practical use.

U.S. Pat. No. 4,817,625 to Miles discloses a self-inductance sensor having a conductor secured to a band of distensible material. The conductor includes two portions each extending from one end of he band to the other and each having a geometric shape such as a sawtooth configuration whereby the two portions in juxtaposition to each other form a series of substantially enclosed geometrically shaped areas. The change in shape of the areas results in a change in the self-inductance of the conductor. The geometric shapes of the conductors eliminates the need for the sensor to encompass the entire circumference of the torso, but limit the flexibility of the sensor, and increase manufacturing time.

U.S. Pat. No. 5,131,399 to Sciarra discloses a transducer apparatus for performing tidal volume measurements on a patient, the volume of air the patient inhales and exhales during respiration, comprising a first inductive transducing means for producing a signal representing size changes in the patient's thoracic region, a second inductive transducing means of producing a signal representing size changes in the patient's abdominal region, and a means for mounting the first and second inductive transducing means in a predetermined spaced relationship corresponding to a distance between transducing positions on the patient's thoracic region and abdominal regions, respectively. The inductive means of the preferred embodiment is described as including a pair of coiled wires wound side-by-side to provide a relatively high mutual inductance, and which form a bifilar transformer which provides tight inductive coupling therebetween. The inductive respiration transducer has a generally elongated, oval configuration so that it extends substantially along the length of the first belt arm. This transducer is limited in its degree of extensibility and necessarily requires a complex and expensive manufacturing process.

U.S. Pat. No. 5,301,678 to Watson et al discloses a stretchable band-type transducer, particularly suited for use with respiration monitoring, having a zig-zag pattern of conductors sandwiched between two strips of elastic material. This transducer remains limited in its degree of extensibility and by its inability to reduce false paradox (polarity shifts).

U.S. Pat. No. 5,913,830 to Miles discloses an inductive plethysmography sensor with a conductor having alternating active and inactive segments. The active segments have a narrow diamond shape which minimizes the possibility of signal artifact - undesirable signal characteristics due to body movement, changes in sleeping position, physical displacement, physical deformation, changes in relative calibration of the chest and abdominal compartments, electrical interference by the chest sensor to the abdominal sensor (and vice-versa), and electrical interference from external electron-magnetic fields including electrical magnetic properties of the torso. Because of the conductor design, the sensors can be placed completely about the chest and abdomen with any overlap arranged so that active segments overlap inactive segments. This addresses some of the sources of artifact to which an inductive transducer is subjected but does not sufficiently address the issue of false paradox.

U.S. Pat. No. 6,461,307 to Kristbjarnarson et al discloses a disposable sensor for measuring respiration that includes at least one flexible ribbon adapted to encircle a portion of a patient (e.g., chest or abdomen). Each flexible ribbon has a conductor strip secured to the ribbon or woven into the ribbon, that extends in a zig-zag or other similar pattern. The disposable sensor also includes a connector assembly for connecting and securing a first free end of the ribbon to a second free end of the ribbon. The connector assembly is operatively coupled to the conductor, and is further adapted to be connected to a monitoring device. This disclosure teaches a transducer that is lacking in durability for other than disposable use.

US 2002/0032388 A1 discloses a disposable sensor for monitoring and measuring the respiration of a patient. The disposable sensor includes at least one flexible woven ribbon adapted to encircle a portion of the patient. The woven flexible ribbon includes a plurality of elastic threads extending in a lengthwise direction. The elastic threads are interconnected by an inelastic thread extending in a widthwise direction. Each flexible ribbon includes a conductor strip secured thereto. The conductor strip extends in a zig-zag or other predetermined pattern. The disposable sensor also includes a connector assembly for connecting and securing a first free end of the ribbon to a second free end of the ribbon.

### OBJECTS OF THE INVENTION

It is an object of the present invention to provide a reusable RIP transducer that overcomes the limitations of the prior state of the art. Another object of the present invention is to provide an extensible RIP transducer for monitoring a patient's respiration having a common size that can be expanded for use on a wide range of patient sizes without loss of signal quality. It is another object of the present invention to provide a low-cost, reusable RIP transducer for monitoring a patient's respiration that can easily be cleaned by common machine washing. It is yet another object of the present invention to provide a low-cost reusable RIP transducer that can be easily applied to a patient. Another object of the present invention is to provide a reusable RIP transducer that can be easily mass-produced. It is another object of the present invention to provide a low-cost reusable RIP transducer that provides an improved signal-to-noise ratio over the prior art.

### SUMMARY OF THE INVENTION

The above-mentioned object is solved according to the present invention by a transducer according to claim 1, a method according to claim 6 and an inductance plethysmograph according to claim 14.

The present invention is a reusable inductance plethysmograph transducer particularly suited for use in respiratory monitoring. The transducer is in the form of a woven fabric providing a substantially flat extensible belt for encircling a portion of a patient for a wide range of patient sizes. The transducer is used for monitoring changes in cross-sectional area corresponding to changes in volume (as measured by changes in cross-sectional area) of an distensible organ such as the patient's chest or abdomen. At least one electrical conductor is woven directly into the fabric in a manner that improves the electrical performance of the transducer over the prior art in two ways. First, a high-density weave is used for the fabric that produces many more inductive turns of the embedded conductor(s), thereby increasing the overall inductance change, thus improving the signal to noise ratio, and increasing the expandability of the effective length of the transducer. Secondly, the conductor(s) are oriented within the weave perpendicular to the surface of the torso of a patient being monitored, thus reducing signal artifact due to body capacitance and allowing for amplitude changes in the inductive loops, further increasing expandability. A single transducer size could therefore be used on a wide range of patient sizes and around various different sized body parts.

At least one electrical contact is provided at each end of the conductor for simple, releasable connection to signal cables for interface with electronic measurement equipment. An attachment means is provided for releasably connecting and securing the ends of the extensible transducer belt about a patient, and is preferably in the form of corresponding plastic buckle ends at each end of the belt.

In addition to improvements in the electrical performance, the manufacture of the inductance sensor is a single step process that can be carried out in mass production on existing looms, reducing the overall cost while improving the flexibility, durability, and ease of use. The present invention is also machine washable making reuse much less labor intensive and therefor much less expensive.

### BRIEF DESCRIPTION OF THE DRAWINGS

- FIG. 1: is a partial plan view of a single-conductor transducer of the present invention.
- FIG. 2: is a partial elevation view of the single-conductor transducer of the present invention.

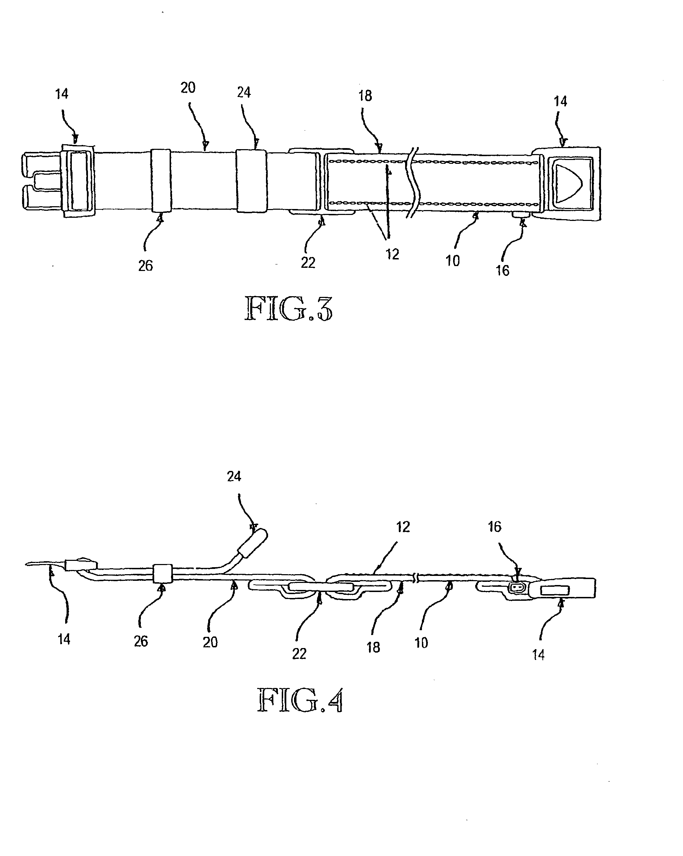

- FIG. 3: is a partial plan view of a two-conductor transducer of the present invention.
- FIG. 4: is a partial elevation view of the two-conductor transducer of the present invention.
- FIG. 5: is a table of comparative respiration waveforms - those obtained using piezo technology versus those obtained using RIP technology, shown as amplitude versus time.

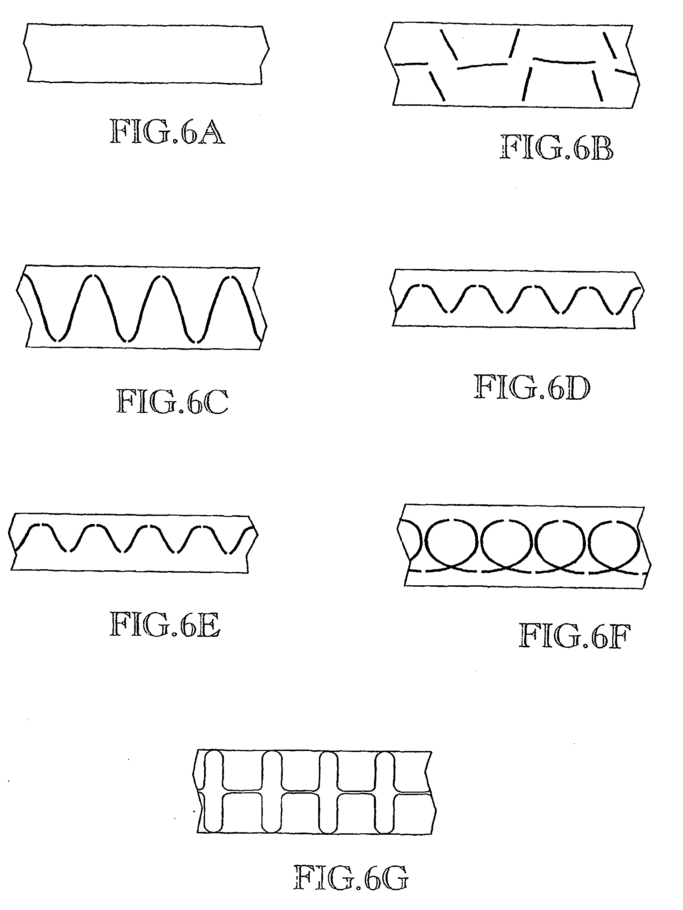

- FIG.6: is a graphic presentation of the test results for tests on various respiratory plethysmograph transducer compared to the transducer of this invention.

### DETAILED DESCRIPTION OF THE INVENTION

For a complete understanding of the features and operation of the present invention, reference is now made to the drawings of the invention along with the accompanying figures in which corresponding numerals in the different figures refer to corresponding parts of the invention. The present invention is generally a reusable transducer having a woven elastic substrate with at least one extremely flexible conductor woven concurrently with the elastic in a plane substantially perpendicular to the surface of a patient's torso. The transducer can be formed in various different embodiments using different numbers and orientation of conductors connected in different ways to the inductance measuring circuitry.

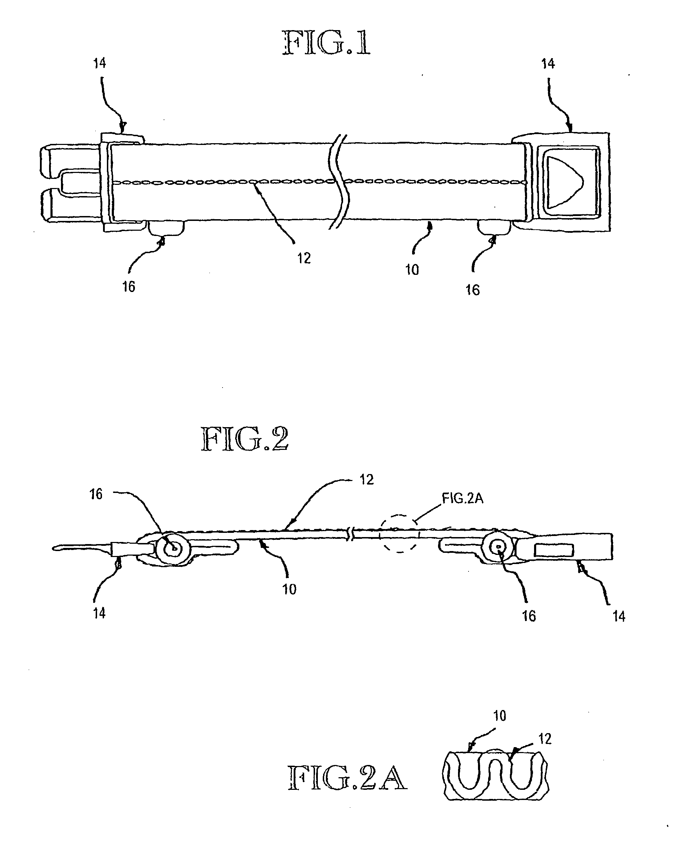

FIG. 1 shows a single conductor respiratory inductive plethysmography (RIP) transducer of the present invention. The transducer is a belt having a woven fabric in the form of an elastic substrate 10 and a single conductor 12 that is woven into the elastic substrate 10 and extends along the entire length of the elastic substrate 10. The ends of the belt are attached to releasable connectors 14 - one for connecting a first end of the belt to a second end to secure the encompassing belt around a body part under study. A set of electrical connectors 16 conductively attached to the conductor 12 ends are located at each end of the belt to facilitate electrical interfacing of the transducer with inductance measurement circuitry. The inductance measurement circuitry could use an LC oscillator with the belt as the inductive element, measuring electrical frequency to determine the inductance of the belt. Small RF transformers located in the circuit in proximity to the transducer, at or in close proximity to the electrical connectors 16, could be provided on one embodiment to magnify the inductance change of the transducer, thereby increasing the signal measurable by the circuitry and also providing a means of electrical isolation for purposes of patient electrical safety. Using transformers close to the transducers allows the use of standard 7' wire sets between the transducer and the measurement circuitry without significant signal degradation. The frequency change could then be converted to a corresponding voltage that would be readable by a standard physiological recording device. An alternate embodiment could use circuitry to measure phase shift of a high frequency signal through the transducer, and the increased phase shift would correspond to an increased inductance.

FIG. 2 shows a side view of the transducer with a close-up view detailing vertical orientation of the conductor 12 woven into the elastic substrate 10 that provides a plurality of perpendicularly oriented inductive turns of the conductor 12 with respect to the surface of a patient's body. Because the conductor 12 is woven directly into the elastic substrate 10 as the belt is being manufactured on a loom, the conductor 12 will have a very high number of inductive turns that corresponds with the density of the weave for the elastic substrate 10. The result is a transducer belt with a high degree of expandability.

Another embodiment of the present invention is a two-conductor design as shown in FIG's. 3 and 4. In this embodiment, the belt is comprised of two segments, a first segment 18 having two conductors 12 woven into the elastic substrate 10, and a second segment 20 having no conductors. This embodiment does not require that the transducer completely surround the circumference of the torso, and thus a non-transducer section is used to complete the circumference. The inductance signal is not based on cross-sectional area of the torso for this embodiment, but is instead based on inductance changes of a loop of the conductor 12 within the elastic substrate 10 of the belt itself. The two rows of conductors 2 are shorted together where the first segment 18 ends and the second segment 20 begins. The first segment 18 is connected to the second segment 20 by a coupler member, about which ends of the first segment 18 and second segment 20 are looped and fastened. An end cap 24 can be provided to dress the free end of the second segment 20 and prevent unraveling. The overall length of the transducer is made even more adjustable by providing a belt loop 26 for the second segment 20 that enables positioning the releasable connector along the length of the second segment 20, and securing the excess length within the belt loop 26. The two-conductor design enables locating the electrical connectors 16 at a common location, preferably as a two-terminal single connector, that allows the transducer to be applied more easily to the patient.

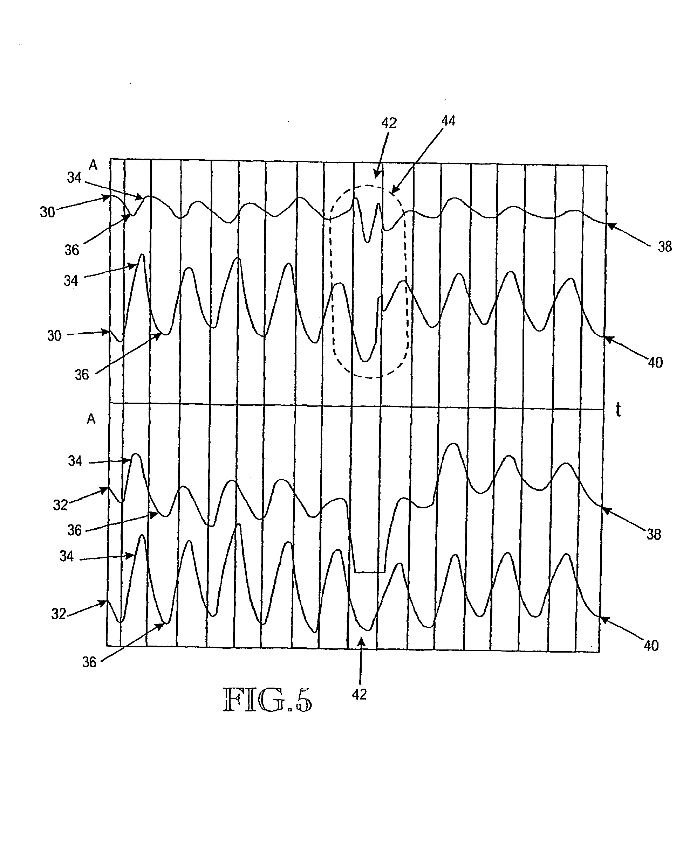

FIG. 5 is a table of comparative respiration waveforms - those obtained using piezo technology shown in the top set of waveforms (piezo waveforms 30), versus those obtained using RIP technology shown in the bottom set of waveforms (RIP waveforms 32). The waveform amplitude is the magnitude of cross-sectional area measured plotted over time. Positive waveform excursions 34 represent an increase in cross-sectional area associated with a patient's inhaling. Negative waveform excursions 36 represent a decrease in cross-sectional area associated with a patient's exhaling. For each waveform set, the upper waveform is a thoracic signal 38, and the lower waveform is an abdominal signal 40. A change in the waveforms is seen in the middle of the time scale at a common time reference 42 resulting from a body position shift. The piezo waveforms 30 clearly show what is referred to as a false paradox signal artifact 44, which shows a change in signal polarity of the thoracic inductance signal 38 to the abdominal inductance signal 40. While a change in waveform morphology is evident in the RIP waveforms 32, no false paradox signal artifact is evident.

### The Present Invention In Use:

In use, a patient being monitored for respiration using a preferred embodiment of the reusable inductive transducer of the present invention would have two belts applied - one around the abdomen, and another around the chest. Two wire sets are connected to a releasable electrical connector on each belt of the patient transducer at one end, and to the measurement electronics at the other. The wire sets are made of tinsel wire in order to provide strength and flexibility. Each wire set is made up of two separate insulated conductors, preferably bound as a single cable, that bifurcate at the cable ends to allow ease of connection to connector locations at the belt ends. A small transformer is disposed within the cable at the point of bifurcation that provides electrical isolation between the patient and the measurement electronics, and to magnify the inductance of the transducer, thus negating the high electrical resistance that is characteristic of the tinsel wire. The monitoring electronics us an LC oscillator to measure the inductance detected from each of the belts. The oscillator converts changes in electrical frequency produced by changes in the inductance of each belt to voltage changes that are measurable by a polysomnographic recording device.

When a patient breaths, each belt will expand and contract with the chest and abdomen. During normal respiration, the chest and abdomen will expand and contract in unison. Inhalation increases the cross-sectional area of the chest and abdomen and creates an increased inductance in each of the belts, which is then processed by the measurement electronics and output to a recording device. Exhalation decreases the cross-sectional area, which creates a decreased inductance that is similarly measured and recorded. If a patient has an airway obstruction, the chest and the abdomen will no longer move in unison, which causes the measured inductance signals to be out of phase with one another. These signals are monitored and output to then be interpreted by a polysomnographic technologist studying the patient. When a patient's airway is totally obstructed, the chest and abdomen will move 180 degrees out of phase (as the chest expands with inspiration, the abdomen contracts). This is referred to as paradoxical breathing, and is the chief identifier of obstructive apnea. A common artifact, or source of error, encountered through use of piezo technology is that changes in a patient's body position can produce false indications of paradoxical breathing, even though the patient is breathing normally.

### Description Of Manufacture:

A method of manufacturing a reusable inductive plethysmographic transducer of the present invention is outlined as follows: The transducer consists of a highly flexible, high strand count copper wire conductor woven into an elastic fabric belt, preferably 1" wide. The elasticity of the fabric is provided by neoprene strands running the length of the belt, around which the fabric and conductor are woven. The wire insulation and fabric are both biocompatible and are intended for sustained contact with living human tissue.

The transducer belt can be woven on a variety of looms commonly known in the art of manufacturing elastic fabrics. The material is woven such that the conductor repeatedly passes through the plane of the belt while continuing through the length of the belt, as shown in FIG. 2. The result is the creation of a continuous sinusoidal wave pattern of conductor wire oriented perpendicularly with respect to the top and bottom surfaces of the belt (as opposed to a parallel or surface plane orientation of conductor wave pattern). Particularly, this effect is achieved by replacing a strand of yarn in a warp on the loom with the conductor wire. The warp threads run the length of the belt, the weft runs transverse to the long axis of the belt and is not elastic. During the weaving process, alternate strands of the warp are pulled apart, and the weft is pulled between them, forming the transverse strands of the fabric. The alternate strands of the warp are then exchanged, wrapping around the weft, and the weft is passed back through. This process is repeated continually for the length of the fabric required. The wire is flexible enough to replace a strand of the yarn in the warp, and is used in the same way as yarn during weaving. This is what gives the conductor wire its sinusoidal wave shape and orientation perpendicular to the plane of the fabric, its high number of wave turns within the fabric, and the flexibility and structure of the surrounding fabric. The functional result is a greatly improved signal-to-noise ratio over RIP transducers of the prior art.

Completion of the manufacturing process includes the steps of cutting the woven belt to length and exposing the ends of the conductor wires. Releasable electrical connectors are then soldered to the conductor wire ends, (such as common 1.5 mm ECG safety connector jacks that are well know in the art of patient monitoring devices), preferably such that the connectors are within close proximity to one another when one end of the belt is secured to the other. The ends of the belt are stitched or thermally welded to prevent unraveling of the woven material. A buckle assembly, such as a plastic snap-type buckle, or other releasable connector means are affixed to each end of the belt to enable the belt's being secured about a patient body part, particularly the chest and abdomen.

Because the RIP transducer of the present invention can be manufactured without significant changes to the loop on which the belt is woven, manufacturing costs can be kept to a minimum and mass production is readily achieved. Because of the improved resilience of the transducer's having a conductor woven into the elastic substrate, as opposed to having a conductor bonded to a belt surface as with several examples of the prior art, the RIP transducer of the present invention is suitable for washing and continued re-use.

### Comparative Analysis Of RIP Transducers:

Seven different respiratory inductance plethysmograph transducers, including a transducer of the present invention, were analyzed using a standardized test procedure.

### Test Plan:

Multiple configurations were created in the attempt to find a belt with the right electrical characteristics, while maintaining manufacturability and keeping costs to a minimum. Electrically, inductance is the most important feature of the belts, particularly the change in inductance during breathing. Resistance is also very important, if the belt is too resistive, the Q of the belt goes down, making any filter or oscillator designed using the belt as the inductive component less precise.

### Test Equipment:

* HP 34401A Multimeter
* AADE L/C Meter IIB

### Test Procedure:

1. The first belt should be placed around the chest of the test subject; the belt should be connected to the L/C Meter in L mode.
2. The test subject should exhale completely, and the inductance value recorded, the test subject should then completely inflate their lungs, and again record the inductance value.
3. Measure the resistance of the belt.
4. Lay the belt flat and measure the length.
5. Stretch the belt until the fabric or the wire becomes taut, and measure the length again.

### Test Report:

| Belt | L min (*µ*H) | L max (*µ*H) | ΔL (*µ*H) | R (Ω) | R(Ω)/L(in.) | Length(in.) | Length max. (in.) | Stretch % |
|---|---|---|---|---|---|---|---|---|
| | | | | | | | | |
| 1 | 2.827 | 2.976 | 0.149 | 1.509 | 0.053 | 28.4 | 52.8 | 186 |
| 2 | 3.745 | 3.785 | 0.040 | 3.330 | 0.088 | 37.9 | 48.6 | 128 |
| 3 | 4.697 | 4.796 | 0.099 | 3.341 | 0.069 | 48.3 | 74.4 | 154 |
| 4 | 2.543 | 2.610 | 0.067 | 1.177 | 0.032 | 37.0 | 47.0 | 127 |
| 5 | 2.666 | 2.728 | 0.062 | 1.665 | 0.045 | 37.2 | 46.1 | 124 |
| 6 | 5.030 | 5.050 | 0.020 | 3.271 | 0.090 | 36.5 | 54.0 | 148 |
| 7 | 2.780 | 2.795 | 0.015 | 2.827 | 0.076 | 37.2 | 49.2 | 132 |

### Notable Occurrences:

* The Belt 4 is 32AWG while Belt 3 and Belt 5 are 34AWG.
* The only belt that wasn't restricted by the wire was Belt 1, which was over the specified Stretch % of the fabric when judged to be at maximum Stretch %.
* Belt 1 has the largest change in inductance per stretch; no other belt is within 30%.
* Belt 1 also has one of the lowest resistances.

### Conclusions:

Electrical: Having more than double the inductance change of any other belt under 2O, Belt 1 has the most desirable characteristics. Of the seven belts tested, only 3 belts were under 20, and all of those were over 1O. One belt was within 50% of the inductance change of Belt 1, and that was Belt 3, but Belt 3 also had the highest resistance of any belt, more than twice that of Belt 1. Several of the other belts had higher natural inductance than Belt 1, but net inductance can be added to with series inductors to achieve minimum oscillation and Q values.

Physical: Belt 1 is nearly a finished product, any other pattern would require at least one additional layer of covering to protect the loose wire from snags and abrasion, making them heavier to wear, and making breathing more difficult. Due to the gauge of the wire required for being stitched or sewn, a sharp pull can separate the copper strands, rendering the belt useless or erratic. Because of the wire orientation in Belt 1, the elastic is the only limiting factor, pull force cannot be applied directly to the wire until the elastic is well past its specified stretch percentage. This is because the elasticity of the webbing in the Z axis allows the Belt 1 wire zigzag to change amplitude, so the wire can almost straighten. Other belts have fixed amplitudes because the webbing is not elastic in the Y axis, which is the orientation of the other belts' oscillations. This can result in sine waves turning into saw tooth waves, endangering the wire when the elastic is stretched. From a manufacturing perspective Belt 1 is still the best choice, two yards of fabric from the supplier costs only a few cents more than 1 minute of manufacturing time, which would be insufficient to produce more than a few stitches of any other pattern.

Visual: Belt 1 has a thin, professional appearance that stands on its own, wire can be colored to accent the belt, or be hidden from view depending on the market. Any accents to other belts would be additional labor and cost.

### INDUSTRIAL APPLICABILITY

The present invention has applicability to transducers for use in the medical field for physiological patient monitoring, specifically for an expandable respiratory inductance plethysmograph transducer particularly suited for use in respiratory monitoring for receiving signals representative of patient breathing.

In compliance with the statute, the invention has been described in language more or less specific as to transducers for use in physiological patient monitoring. It is to be understood, however, that the invention is not limited to the specific means or features shown or described, since the means and features shown or described comprise preferred ways of putting the invention into effect.

Additionally, while this invention is described in terms of being used for patient respiratory monitoring in the medical field, it will be readily apparent to those skilled in the art that the invention can be adapted to other uses including, but not limited to, other fields in the life sciences and related research industries, and therefore the invention should not be construed as being limited to respiratory monitoring. The invention is, therefore, claimed in any of its forms or modifications within the legitimate and valid scope of the appended claims, appropriately interpreted in accordance with the doctrine of equivalents. construed as being limited to respiratory monitoring. The invention is, therefore claimed in any of its forms or modifications within the legitimate and valid scope of the appended claims, appropriately interpreted in accordance with the doctrine of equivalents.

## Claims

1. A transducer for monitoring changes in cross-sectional area of a distensible organ of a patient, the transducer comprising:
at least one flexible extensible member (10) having a substantially flat elongated surface for encircling a portion of the patient, a first free end, and a second free end;
at least one electrical conductor (12) disposed within the flexible extensible member (10) and extending substantially lengthwise along the elongated surface, the conductor (12) having a first end and a second end;
at least one electrical contact (16) located at each of the first end and the second end of the conductor (12) for providing an interface for electronic monitoring circuitry; and
an attachment assembly (14) for releasably connecting and securing the first free end to the second free end, whereby
the at least one extensible member (10) is encircled about the distensible organ of the patient for obtaining inductive signals corresponding to changes in cross-sectional area of the distensible organ,
**characterized in that** the at least one electrical conductor (12) comprises a plurality of inductive turns repeatedly passing through the substantially flat elongated surface of the at least one flexible extensible member (10), wherein a plane in which the inductive turns are arranged is oriented substantially perpendicular to the substantially flat elongated surface.

2. The transducer of Claim 1, wherein the distensible organ of the patient is a thorax and the monitored changes in cross-sectional area of the thorax correspond to respiration.

3. The transducer of Claim 1, wherein the distensible organ of the patient is an abdomen and the monitored changes in cross-sectional area of the abdomen correspond to respiration.

4. The transducer of Claim 1, wherein the at least one flexible extensible member (10) is comprised of a woven fabric and the at least one electrical conductor (12) is woven into the fabric.

5. The transducer of Claim 1, wherein said electrical conductor (12) isarranged in a substantially sinusoidal wave pattern.

6. A method of monitoring changes in cross-sectional area of a distensible organ of a patient, the method comprising the steps of:
providing an extensible inductance plethysmograph transducer having at least one flexible extensible member (10) comprising:
a substantially flat elongated surface for encircling a portion of the patient, a first free end, and a second free end;
at least one electrical conductor (12) disposed within the flexible extensible member (10) and extending substantially lengthwise along the elongated surface, the conductor (12) having a first end and a second end;
at least one electrical contact (16) located at each of the first end and the second end of the conductor (12) for providing an interface for
electronic monitoring circuitry; and
an attachment assembly (14) for releasably connecting and securing the first free end to the second free end;
encircling the inductance plethysmograph transducer about the distensible organ of the patient, whereby the flat elongated surface of the inductance plethysmograph transducer engages an outer surface of the distensible organ;
connecting the attachment assembly (14) to secure the inductance plethysmograph transducer in position about the distensible organ of the patient;
engaging the at least one electrical contact (16) with an electronic interface that communicates with electronic monitoring circuitry for providing output indicative of changes in the cross-sectional area of the distensible organ of the patient,
**characterized in that** the at least one electrical conductor (12) comprises a plurality of inductive turns repeatedly passing through the substantially flat elongated surface of the at least one flexible extensible member (10), wherein a plane in which the inductive turns are arranged is oriented substantially perpendicular to the substantially flat elongated surface.

7. The method of claim 6, wherein the distensible organ is the patient's thorax and the monitored changes correspond to the patient's respiration.

8. The method of claim 6, further comprising the step of providing a transformer at the electronic interface that electrically engages the transducer for magnifying inductance change detected by the transducer, thereby increasing the signal measurable by the electronic monitoring circuitry.

9. The method of Claim 6, wherein said transducer is formed form a woven belt cut to length to expose conductor wires imbedded therein is the weaving process used to form said belt.

10. The method of Claim 9, wherein said conductor exposed by cutting said belt are connected to means to monitor inductance.

11. The method of Claim 6, wherein said transducer is connected to an inductance measurement device using releaseable conductors.

12. The method of Claim 6, wherein said transducer is positioned around the abdomen of the patient.

13. The method of Claim 6, wherein said transducer is composed of a first and a second extendible member (10, 20) connected end to end, said first extendible member (10) having said conduction means imbedded therein.

14. An inductance plethysmograph suited for use in respiration monitoring comprising a transducer as defined in claim 1.

15. The inductance plethysmograph of Claim 14 wherein said transducer is in the form of a woven fabric belt, and further having two electrical conductors (12) woven into said belt.

## Patentansprüche

1. Wandler zum Überwachen von Änderungen der Querschnittfläche eines dehnbaren Organs eines Patienten, wobei der Wandler Folgendes umfasst:
mindestens ein flexibles ausdehnbares Element (10) mit einer im Wesentlichen flachen, länglichen Oberfläche zum Einkreisen eines Teils des Patienten, einem ersten freien Ende und einem zweiten freien Ende;
mindestens einen elektrischen Leiter (12), der innerhalb des flexiblen ausdehnbaren Elements (10) angeordnet ist und im Wesentlichen in Längsrichtung entlang der länglichen Oberfläche verläuft, wobei der Leiter (12) ein erstes Ende und ein zweites Ende hat;
mindestens einen elektrischen Kontakt (16), der sich auf jeweils dem ersten Ende und dem zweiten Ende des Leiters (12) befindet, um eine Schnittstelle für die elektronische Überwachungsschaltung zu schaffen; und
eine Befestigungsbaugruppe (14) zum lösbaren Verbinden und Sichern des ersten freien Endes mit dem zweiten freien Ende, wobei
das mindestens eine ausdehnbare Element (10) das dehnbare Organ des Patienten umgibt, um induktive Signale zu erhalten, die Änderungen der Querschnittfläche des dehnbaren Organs entsprechen,
**dadurch gekennzeichnet, dass** der mindestens eine elektrische Leiter (12) eine Vielzahl von induktiven Wicklungen umfasst, die wiederholt durch die im Wesentlichen flache, längliche Oberfläche des mindestens einen flexiblen ausdehnbaren Elements (10) verlaufen, wobei eine Ebene, in der die induktiven Wicklungen angeordnet sind, im Wesentlichen senkrecht zu der im Wesentlichen flachen, länglichen Oberfläche ausgerichtet ist.

2. Wandler nach Anspruch 1, wobei das dehnbare Organ des Patienten ein Thorax ist und die überwachten Änderungen der Querschnittfläche des Thorax der Atmung entsprechen.

3. Wandler nach Anspruch 1, wobei das dehnbare Organ des Patienten das Abdomen ist und die überwachten Änderungen der Querschnittfläche des Abdomens der Atmung entsprechen.

4. Wandler nach Anspruch 1, wobei das mindestens eine flexible ausdehnbare Element (10) aus einem gewebten Stoff besteht und der mindestens eine elektrische Leiter (12) in den Stoff eingewebt ist.

5. Wandler nach Anspruch 1, wobei der genannte elektrische Leiter (12) in einem im Wesentlichen sinuswellenförmigen Muster angeordnet ist.

6. Verfahren zum Überwachen von Änderungen der Querschnittfläche eines dehnbaren Organs eines Patienten, wobei das Verfahren die folgenden Schritte umfasst:
Schaffen eines ausdehnbaren Induktivitätsplethysmograph-Wandlers mit mindestens einem flexiblen ausdehnbaren Element (10), das Folgendes umfasst:
eine im Wesentlichen flache, längliche Oberfläche zum Einkreisen eines Teils des Patienten,
ein erstes freies Ende und ein zweites freies Ende;
mindestens einen elektrischen Leiter (12), der innerhalb des flexiblen ausdehnbaren Elements (10) angeordnet ist und im Wesentlichen in Längsrichtung entlang der länglichen Oberfläche verläuft, wobei der Leiter (12) ein erstes Ende und ein zweites Ende hat;
mindestens einen elektrischen Kontakt (16), der sich auf jeweils dem ersten Ende und dem zweiten Ende des Leiters (12) befindet, um eine Schnittstelle für die elektronische Überwachungsschaltung zu schaffen; und
eine Befestigungsbaugruppe (14) zum lösbaren Verbinden und Sichern des ersten freien Endes mit dem zweiten freien Ende;
Umgeben des dehnbaren Organs des Patienten mit dem Induktivitätsplethysmograph-Wandler, wobei die flache, längliche Oberfläche des Induktivitätsplethysmograph-Wandlers mit einer Außenfläche des dehnbaren Organs zusammenwirkt;
Verbinden der Befestigungsbaugruppe (14) zum Sichern des Induktivitätsplethysmograph-Wandlers in der Position um das ausdehnbare Organ des Patienten herum;
Einrasten des mindestens einen elektrischen Kontakts (16) in eine elektronische Schnittstelle, die mit der elektronischen Überwachungsschaltung kommuniziert, um ein Ausgangssignal zu liefern, das die Änderungen der Querschnittfläche des dehnbaren Organs des Patienten angibt,
**dadurch gekennzeichnet, dass** der mindestens eine elektrische Leiter (12) eine Vielzahl von induktiven Wicklungen umfasst, die wiederholt durch die im Wesentlichen flache, längliche Oberfläche des mindestens einen flexiblen ausdehnbaren Elements (10) verlaufen, wobei eine Ebene, in der die induktiven Wicklungen angeordnet sind, im Wesentlichen senkrecht zu der im Wesentlichen flachen, länglichen Oberfläche ausgerichtet ist.

7. Verfahren nach Anspruch 6, wobei das dehnbare Organ der Thorax des Patienten ist und die überwachten Änderungen der Atmung des Patienten entsprechen.

8. Verfahren nach Anspruch 6, weiterhin umfassend den Schritt des Bereitstellens eines Transformators an der elektronischen Schnittstelle, der mit dem Wandler elektrisch zusammenwirkt, um die durch den Wandler detektierte Induktivitätsveränderung zu vergrößern, wodurch das durch die elektronische Überwachungsschaltung messbare Signal erhöht wird.

9. Verfahren nach Anspruch 6, wobei der genannte Wandler aus einem gewebtem Riemen gebildet wird, der auf Länge geschnitten wird, um die darin eingebetteten Leiterdrähte freizulegen, wobei der Webevorgang genutzt wird, um den genannten Riemen zu bilden.

10. Verfahren nach Anspruch 9, wobei die genannten Leiter, die durch Abschneiden des genannten Riemens freigelegt wurden, mit Mitteln zum Überwachen der Induktivität verbunden werden.

11. Verfahren nach Anspruch 6, wobei der genannte Wandler mittels lösbaren Leitern mit einer Vorrichtung zur Messung der Induktivität verbunden wird.

12. Verfahren nach Anspruch 6, wobei der genannte Wandler um das Abdomen des Patienten herum positioniert wird.

13. Verfahren nach Anspruch 6, wobei der genannte Wandler zusammengesetzt ist aus einem ersten und einem zweiten ausdehnbaren Element (10, 20), deren Enden miteinander verbunden sind, wobei in das erste ausdehnbare Element (10) die genannte Leitungsmittel eingebettet sind.

14. Induktivitätsplethysmograph zur Verwendung bei der Überwachung der Atmung, wobei der Induktivitätsplethysmograph einen Wandler nach Anspruch 1 umfasst.

15. Induktivitätsplethysmograph nach Anspruch 14, wobei der genannte Wandler in der Form eines gewebten Stoffriemens vorliegt und weiterhin zwei elektrische Leiter (12) aufweist, die in den Riemen eingewebt sind.

## Revendications

1. Transducteur pour surveiller les changements dans la surface en coupe d'un organe dilatable d'un patient, le transducteur comprenant :
au moins un élément extensible flexible (10) ayant une surface allongée sensiblement plane pour entourer une partie du patient, une première extrémité libre et une seconde extrémité libre ;
au moins un conducteur électrique (12) disposé à l'intérieur de l'élément extensible flexible (10) et s'étendant sensiblement longitudinalement le long de la surface allongée, le conducteur (12) ayant une première extrémité et une seconde extrémité ;
au moins un contact électrique (16) situé au niveau de chacune de la première extrémité et de la seconde extrémité du conducteur (12) pour fournir une interface pour les circuits de surveillance électroniques ; et
un ensemble de fixation (14) pour relier et immobiliser de manière amovible la première extrémité libre sur la seconde extrémité libre, moyennant quoi
l'au moins un élément extensible (10) est entouré autour de l'organe dilatable du patient pour obtenir des signaux inductifs correspondant aux changements dans la surface en coupe de l'organe dilatable,
**caractérisé en ce que** l'au moins un conducteur électrique (12) comprend une pluralité de spires inductives passant de façon répétée à travers la surface allongée sensiblement plane de l'au moins un élément extensible flexible (10), dans lequel un plan où sont agencées les spires inductives, est orienté sensiblement perpendiculairement à la surface allongée sensiblement plane.

2. Transducteur selon la revendication 1, dans lequel l'organe dilatable du patient est un thorax et les changements surveillés dans la surface en coupe du thorax correspondent à la respiration.

3. Transducteur selon la revendication 1, dans lequel l'organe dilatable du patient est un abdomen et les changements surveillés dans la surface en coupe de l'abdomen correspondent à la respiration.

4. Transducteur selon la revendication 1, dans lequel l'au moins un élément extensible flexible (10) comprend un tissu tissé et l'au moins un conducteur électrique (12) est tissé dans le tissu.

5. Transducteur selon la revendication 1, dans lequel ledit conducteur électrique (12) est agencé selon un motif d'onde sensiblement sinusoïdale.

6. Procédé de surveillance de changements dans la surface en coupe d'un organe dilatable d'un patient, le procédé comprenant les étapes suivantes :
la fourniture d'un transducteur de pléthysmographe par inductance extensible ayant au moins un élément extensible flexible (10) comprenant :
une surface allongée sensiblement plane pour entourer une partie du patient,
une première extrémité libre et une seconde extrémité libre ;
au moins un conducteur électrique (12) disposé à l'intérieur de l'élément extensible flexible (10) et s'étendant sensiblement longitudinalement le long de la surface allongée, le conducteur (12) ayant une première extrémité et une seconde extrémité ;
au moins un contact électrique (16) situé au niveau de chacune de la première extrémité et de la seconde extrémité du conducteur (12) pour fournir une interface pour les circuits de surveillance électronique ; et
un ensemble de fixation (14) pour relier et immobiliser de manière amovible la première extrémité libre sur la seconde extrémité libre ;
l'encerclement par le transducteur de pléthysmographe par inductance de l'organe dilatable du patient, moyennant quoi la surface allongée plane du transducteur de pléthysmographe par inductance se met en prise avec une surface externe de l'organe dilatable ;
la liaison de l'ensemble de fixation (14) pour immobiliser le transducteur de pléthysmographe par inductance en position autour de l'organe dilatable du patient ;
la mise en prise de l'au moins un contact électrique (16) avec une interface électronique qui communique avec les circuits de surveillance électronique pour fournir une sortie indicative des changements dans la surface en coupe de l'organe dilatable du patient,
**caractérisé en ce que** l'au moins un conducteur électrique (12) comprend une pluralité de spires inductives passant de manière répétée à travers la surface allongée sensiblement plane de l'au moins un élément extensible flexible (10), dans lequel un plan où sont agencées les spires inductives est orienté sensiblement perpendiculairement à la surface allongée sensiblement plane.

7. Procédé selon la revendication 6, dans lequel l'organe dilatable est le thorax du patient et les changements surveillés correspondent à la respiration du patient.

8. Procédé selon la revendication 6, comprenant en outre l'étape de fourniture d'un transformateur au niveau de l'interface électronique qui active électriquement le transducteur pour amplifier le changement d'inductance détecté par le transducteur, améliorant ainsi le signal mesurable par les circuits de surveillance électroniques.

9. Procédé selon la revendication 6, dans lequel ledit transducteur est formé à partir d'une ceinture tissée coupée en longueur pour exposer les fils conducteurs logés à l'intérieur, et dans lequel le procédé de tissage est utilisé pour former ladite ceinture.

10. Procédé selon la revendication 9, dans lequel lesdits conducteurs exposés en coupant ladite ceinture sont connectés aux moyens pour surveiller l'inductance.

11. Procédé selon la revendication 6, dans lequel ledit transducteur est connecté à un dispositif de mesure d'inductance au moyen de conducteurs amovibles.

12. Procédé selon la revendication 6, dans lequel ledit transducteur est positionné autour de l'abdomen du patient.

13. Procédé selon la revendication 6, dans lequel ledit transducteur est composé d'un premier et d'un second éléments extensibles (10, 20) connectés bout à bout, ledit premier élément dilatable (10) ayant lesdits moyens de conduction logés à l'intérieur.

14. Pléthysmographe par inductance adéquat pour une utilisation dans la surveillance respiratoire, comprenant un transducteur selon la revendication 1.

15. Pléthysmographe par inductance selon la revendication 14, dans lequel ledit transducteur se présente sous la forme d'une ceinture en tissu tissé et ayant en outre deux conducteurs électriques (12) tissés dans ladite ceinture.
